# EUROPEAN PATENT APPLICATION

(11) **EP 3 714 852 A1**
(43) Date of publication of application: **30.09.2020**
(21) Application number: 19205614.1
(22) Date of filing: 28.10.2019
(51) Int. Cl.: A61F 13/20, A61F 13/34

(54) **TAMPON WITH MEMBRANE**

(30) Priority: 25.03.2019 TW 108110329
(71) Applicant: Lin, Chung-Sing, Tainan 710 (TW)
(72) Inventor: Lin, Chung-Sing, Tainan 710 (TW)
(74) Representative: Brown, Michael Stanley

(57) **Abstract**

A tampon 1 includes a membrane 10, an inner absorber 20 and a support tube 30. The membrane 10 mounted to the inner absorber 20 and the support tube 30. The inner absorber 20 is located at the center of the tampon 1 and peripherally wrapped by the inner portion 10c of the membrane 10. The support tube 30 is located between the outer portion 10a and inner portion 10c of the membrane. The cord 11 connected to the membrane 10 extends beyond the tampon 1. The membrane 20 contacts the vaginal wall and provides comfort when using the tampon 1.

## Description

### BACKGROUND OF THE INVENTION

### 1. Fields of the invention

The present invention relates to a female product, and more particularly, to a tampon with an applicator to provide comfort using experience.

### 2. Descriptions of Related Art

The time during menstruation for female, the periodic blood that flows as a discharge from the uterus. The time during which menstruation occurs is referred to as menses. The menses occurs at approximately 4 week intervals to compose the menstrual cycle. Sanitary pads and tampons are used during the menstruation to absorb the menstrual flow or sometime the menstrual clots.

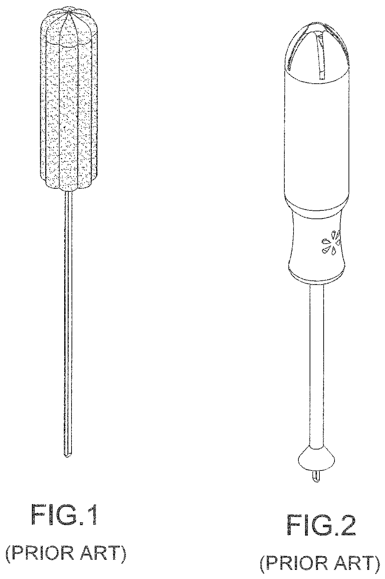

The conventional tampons are cataloged into two types, as shown in Fig. 1, the known first type includes a body and a cord connected to the body. The body is made of compressed high-density cotton. There are multiple radially grooves formed in the outside of the body to ensure that the body expand evenly. The body includes a rounded top end, and the cord is connected to the tail end of the body. The body is inserted into the vagina directly by the user's hand. The cord is used to remove the body from the user's body. As shown in Fig. 2, the second type of the tampons includes a penetrator in which the body is accommodated, and a hollow plunge tube which is connected to the penetrator. The penetrator includes multiple cut-outs which form multiple petal-like plates. The cord extends beyond the plunge tube. When in use, the penetrator is inserted into the vagina and the user pushes the plunger tube toward the penetrator so that the body pushes the petal-like plates outward until the body is sent to the desired position. The penetrator and the plunger tube are then removed from the vagina.

The body of the absorbed will be stayed in the user's body for hours to absorb the menstrual flow. In other words, the body of the absorber is in contact with the vaginal wall for hours. For sanitation reasons, the body of the absorber are compressed and de-hydrated, so that the outer surface of the body will be a little bit rough, and the rough outer surface of the body of the absorber causes uncomfortable feel when inserting the body of the absorber into the vagina. In addition, when the body of the absorber absorbs the menstrual flow and is to be replaced with a new one, some fluid or the menstrual clots may not be able to be absorbed by the body of the absorber. The tampon of the present eliminates the shortcomings of the conventional tampons and includes a membrane, an inner absorber and a support tube. The membrane mounted to the inner absorber and the support tube. The inner absorber is located at the center of the tampon and peripherally wrapped by the inner portion of the membrane. The support tube is located between the outer portion and inner portion of the membrane. The cord connected to the membrane extends beyond the tampon. The membrane contacts the vaginal wall and provides comfort when using the tampon.

### SUMMARY OF THE INVENTION

The present invention relates to a tampon comprises a membrane which is an elongate pocket and made of flexible and expandable material. The membrane includes a closed top end and an opened bottom which includes an opening. Multiple flanges extend outward from the outer surface of the lower portion of the membrane. Each flange is a collar-shaped with a tapered outer surface. A cord made of the same material as the membrane extends from the inner bottom of the closed top end of the membrane An inner absorber has a first end and a second end, wherein the first end of the inner absorber is a rounded end. The inner absorber is made of compressed high-density of cotton. The length of the inner absorber is longer than one half length of the membrane. The cross sectional area diameter of the inner absorber is larger than the inner diameter of the membrane. The inner absorber is axially located in the membrane. The closed top end of the membrane contacts the second end of the inner absorber. The first end of the inner absorber protrudes beyond the membrane. A support tube is a tubular part with two opened ends. The support tube is made of compressed high-density of cotton. The inner diameter of the support tube is larger than the cross sectional area diameter of the inner absorber. The membrane is mounted to the outer absorber from the second end of the membrane. The top end of the membrane and the inner absorber are located in the support tube.

Preferably, the thickness of the membrane is between 0.02 and 0.07 mm.

Preferably, lubricant is applied to the outer portion of the membrane.

The advantages of the present invention are that the outer portion of the membrane contacts the vaginal wall when using the tampon of the present invention. Because the membrane is made of soft material so that when the outer portion of the membrane including the flanges is in contact with the user's vaginal wall, the user does not feel uncomfortable. Besides, the tapered flanges on the membrane are helpful to expand the user's vagina to easily insert the tampon into the user's body.

There are lubricants applied on the outer portion of the membrane including the flanges so that the insertion is smooth and easy without discomfort. The users may apply lubricant to membrane of the tampon if there is no lubricant on the tampon when purchased.

When inserting the inner absorber into the vagina, the outer portion of the membrane contacts the vaginal wall to prevent fluid from leaking even when heavier menstrual flow occurs. The flanges of the membrane also help positioning of the inner in the vagina.

The top end of the inner absorber of the present invention is exposed so that the menstrual flow is immediately absorbed by the inner absorber when inserting the inner absorber into the vagina. When the inner absorber expands, the membrane has good flexibility and extensibility so as to allow the expansion of the inner absorber. The support tube can also be deformed and curved according to the expansion of the inner absorber because the support tube is made of cotton material.

The user pulls the cord outward to remove the inner absorber out, the flanges of the membrane also help to remove the fluid and/or the menstrual clots from the vaginal wall.

The present invention will become more obvious from the following description when taken in connection with the accompanying drawings which show, for purposes of illustration only, a preferred embodiment in accordance with the present invention.

### BRIEF DESCRIPTION OF THE DRAWINGS

- Fig. 1: shows the first type of the conventional tampon;
- Fig. 2: shows the second type of the conventional tampon;
- Fig. 3: shows the tampon of the present invention;
- Fig. 4: is an exploded and cross sectional view of the tampon of the present invention;
- Figs. 5 to 7: show the steps of assembly of the tampon of the present invention;
- Fig. 8: is a cross sectional view of the tampon of the present invention;
- Fig. 9: shows that the tampon of the present invention is packed in a packing film, and
- Fig. 10: shows that the tampon of the present invention is packed in a packing cell.

### DETAILED DESCRIPTION OF THE PREFERRED EMBODIMENT

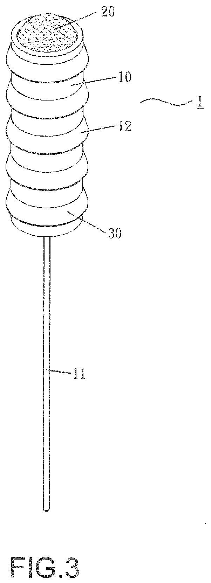

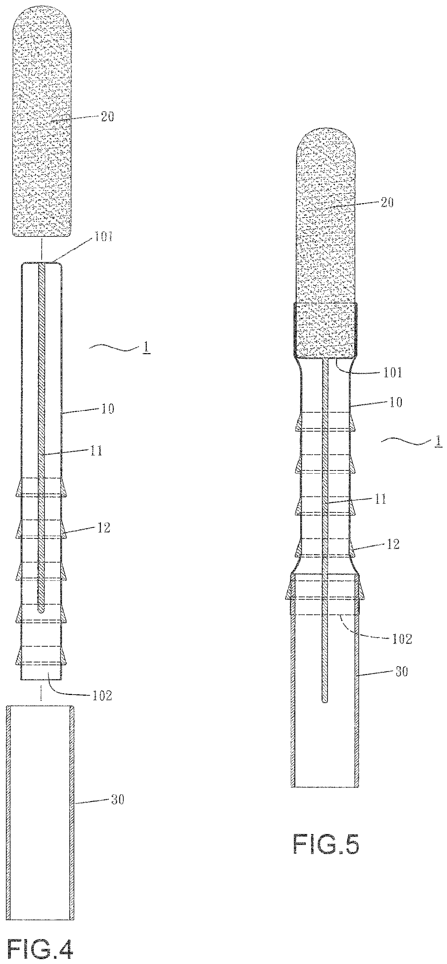

Referring to Figs. 3 and 4, the tampon 1 of the present invention comprises a membrane 10, an inner absorber 20 and a support tube 30. The membrane 10 is an elongate pocket and made of flexible and expandable material such as emulsion and silicone or any proper compound material. The membrane 10 includes a closed top end 101 and an opened bottom which includes an opening 102. A cord 11 made of the same material as the membrane 10 extends from an inner bottom of the closed top end 101 of the membrane 10. Multiple flanges 12 extend outward from the outer surface of the lower portion (close to the opened bottom) of the membrane 10. As shown In Figs. 3 and 4, each flange 12 is a collar-shaped with a tapered outer surface which includes a small top end and a wide bottom end as shown in Figs. 3 and 4. The inner absorber 20 includes a first end and a second end, wherein the first end of the inner absorber 20 is a rounded end. The inner absorber 20 is made of compressed high-density of cotton so as to expand when absorbing fluid. The length of the inner absorber 20 is longer than one half length of the membrane 10. The cross sectional area diameter of the inner absorber 20 is larger than the inner diameter of the membrane 10. The support tube 30 is a tubular part with two opened ends. The support tube 30 is made of compressed high-density of cotton. The inner diameter of the support tube 30 is larger than the cross sectional area diameter of the inner absorber 20.

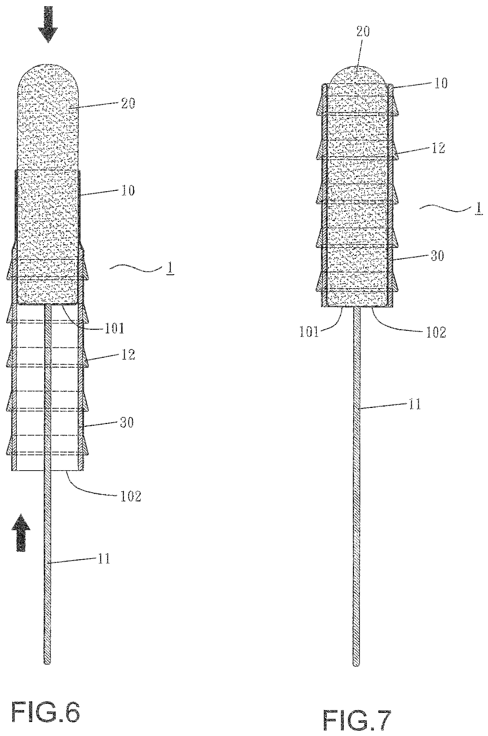

When assembling the tampon 1, as shown in Fig. 5, the two ends of the membrane 10 are expanded by a proper device (not shown), and the support tube 30 is inserted into the lower portion of the membrane 10 from the opening 102 of the opened bottom of the membrane 10 so that the membrane 10 is expanded by the support tube 30 and inserted into the lower portion of the membrane 10. The inner absorber 20 is axially pushes the closed top end 101 of the membrane 10 downward so as to be axially located in the membrane 10, and the second end of the inner absorber 20 reaches the top end of the support tube 30. As shown in Figs. 6 and 7, the inner absorber 20 is continuously pushed downward until the second end of the inner absorber 20 is flushed with the lower end of the support tube 30. The first end of the inner absorber 20 protrudes beyond the membrane 10 as shown in Fig. 3.

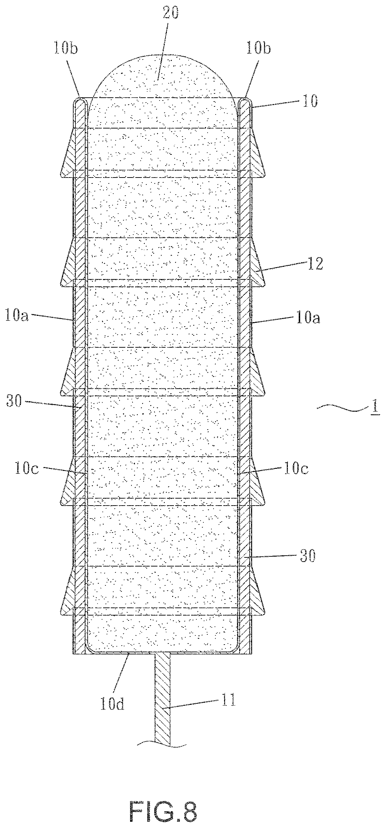

As shown in Fig. 8, when the tampon 1 is assembled, because the support tube 30 is pushed upward from the opening 102 of the membrane 10, and the inner absorber 20 pushes the top end 101 of the membrane 10 downward, the membrane 10 includes a tubular outer portion 10a whose top portion is folded inward and downward to form a top portion 10b, and a tubular inner portion 10c extends from the top portion 10b. A bottom portion 10d is formed and located opposite to the top end 101 of the membrane 10.

As shown in Figs. 7 and 8, the first end of the inner absorber 20 is exposed, and the peripheral portion of the inner absorber 20 is tightly wrapped by the inner portion 10c of the membrane 10, and the second end of the inner absorber 20 contacts the top end 101. The inner portion 10c of the membrane 10 is sandwiched between the inner wall of the support tube 30 and outer peripheral portion of the inner absorber 20. The outer portion 10a of the membrane 10 warps the outer peripheral portion of the support tube 30. The top edge of the outer tube 30 contacts the top portion 10b of the membrane 10. The flanges 12 are formed on outside of the outer portion 10a of the membrane 10. It is noted that the length of the inner absorber 20 is not necessarily the same as the length of the support tube 30. At least two third of the length of the inner absorber 20 is located within the space formed by the support tube 30.

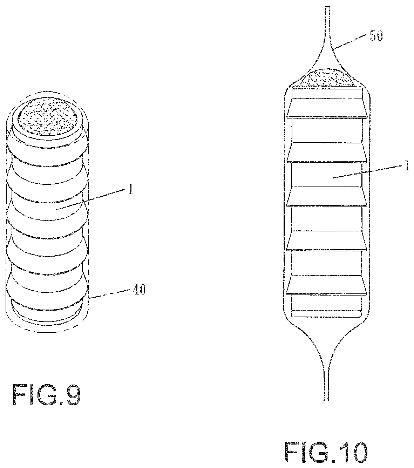

As shown in Fig. 9, the tampon 1 of the present invention is packed in a packing film 40 which can be a shrink film to protect the tampon 1 from being contaminated. Alternatively, the tampon 1 of the present invention can also be packed in a packing cell 50 as shown in Fig. 10. The outer portion 10a of the membrane 10 including the flanges 12 can be applied suitable amount of lubricant before being packed so that when the packing material is removed, the applicant is exposed on the outer portion 10a of the membrane 10 including the flanges 12.

The tampon 1 of the present invention can be directly inserted into the user's vagina by hand until the tampon 1 reaches a desired position close to the cervix, or the tampon 1 can be accommodated in a penetrator to be used as an applicator tampon. In other words, the use of the tampon 1 of the present invention is the same as the existed tampons.

The advantages of the present invention are that the outer portion 10a of the membrane 10, including the flanges 12, contacts the vaginal wall when using the tampon of the present invention. Because the membrane 10 is made of soft material so that when the outer portion 10a of the membrane 10 including the flanges 12 is in contact with the user's vaginal wall, the user does not feel uncomfortable. Besides, the tapered flanges 12 on the outer portion 10a of the membrane 10 are helpful to expand the user's vagina to easily insert the tampon 1 into the user's body.

There are lubricants applied on the outer portion 10c of the membrane 10 including the flanges 12 so that the insertion is smooth and easy without discomfort. The users may apply lubricant to membrane 10 of the tampon 1 if there is no lubricant on the tampon 1 when purchased. The manufacturers may attached the lubricant, or the users may purchase the lubricant by themselves.

When inserting the inner absorber 20 into the vagina, the outer portion 10c of the membrane 10 contacts the vaginal wall to prevent fluid from leaking even when heavier menstrual flow occurs. The flanges 12 of the membrane also help positioning of the inner absorber 20 in the vagina.

The first end or the top end of the inner absorber 20 of the present invention is exposed so that the menstrual flow is immediately absorbed by the inner absorber 20 when inserting the inner absorber 20 into the vagina. When the inner absorber 20 expands, the membrane 10 has good flexibility and extensibility so as to allow the expansion of the inner absorber 20. The support tube 30 can also be deformed and curved according to the expansion of the inner absorber 20 because the support tube 30 is made of cotton material. The support tube 30 maintains the tampon 1 to be a fixed shape before being used. Even if the support tube 30 is broken due to severe expansion of the inner absorber 20 during use, because the outer portion 10a including the flanges 12 are in contact with the vaginal wall, and the expansion of the inner absorber 20 pushes the support tube 30 to contact against the vaginal wall, so that the function of the inner absorber 20 is not affected.

When the users want to replace a new tampon 1, the user pulls the cord 11 outward to remove the inner absorber 20 out from the vagina, the flanges 12 of the membrane 10 also help to remove the fluid and/or the menstrual clots from the vaginal wall.

The present invention provides a tampon 1 that uses a membrane 10 to wrap the inner absorber 20 and the support tube 30. The inner absorber 20 absorbs the fluid and the membrane 10 helps the removal or insertion of the tampon 1 and reduces the discomfort to the users.

While we have shown and described the embodiment in accordance with the present invention, it should be clear to those skilled in the art that further embodiments may be made without departing from the scope of the present invention.

## Claims

1. A tampon 1 comprising, **in characterized that**
a membrane 10 being an elongate pocket and made of flexible and expandable material, the membrane 10 including a closed top end 101 and an opened bottom which includes an opening 102, multiple flanges 12 extending outward from an outer surface of a lower portion of the membrane 10, each flange 12 being a collar-shaped with a tapered outer surface, a cord 11 made of the same material as the membrane 10 extending from an inner bottom of the closed top end 101 of the membrane 10;
an inner absorber 20 having a first end and a second end, the first end of the inner absorber 20 being a rounded end, the inner absorber 20 made of compressed high-density of cotton, a length of the inner absorber 20 being longer than a half length of the membrane 10, a cross sectional area diameter of the inner absorber 20 being larger than an inner diameter of the membrane 10, the inner absorber 20 axially located in the membrane 10, the closed top end 101 of the membrane 10 contacting the second end of the inner absorber 20, the first end of the inner absorber 20 protruding beyond the membrane 10, and
a support tube 30 being a tubular part with two opened ends, the support tube 30 made of compressed high-density of cotton, an inner diameter of the support tube 30 being larger than the cross sectional area diameter of the inner absorber 20, the membrane 10 mounted to the outer absorber from the second end of the membrane 10, the top end of the membrane 10 and the inner absorber 20 located in the support tube 30.
